# FASCICULE DE BREVET EUROPEEN

(11) **EP 2 997 349 B1**
(45) Date de publication et mention de la délivrance du brevet: **15.04.2020**
(21) Numéro de dépôt: 14725987.3
(22) Date de dépôt: 19.05.2014
(51) Int. Cl.: G01N 21/47, C12Q 1/18, C12Q 1/02

(54) **PROCEDE D'OBSERVATION D'ORGANISMES ET SYSTEME ASSOCIE**
VERFAHREN ZUR BEOBACHTUNG VON ORGANISMEN UND ZUGEHÖRIGES SYSTEM
METHOD FOR OBSERVING ORGANISMS AND ASSOCIATED SYSTEM

(30) Priorité: 17.05.2013 FR 1354476
(43) Date de publication de la demande: 23.03.2016
(73) Titulaire: Commissariat à l'Énergie Atomique et aux Énergies Alternatives, 75015 Paris (FR); Biomérieux, 69280 Marcy l'Étoile (FR)
(72) Inventeur: MARCOUX, Pierre, F-38120 Saint Egreve (FR); DUPOY, Mathieu, F-38500 COUBLEVIE (FR)
(74) Mandataire: Lavoix
(86) Numéro de dépôt international: PCT/EP2014/060263
(87) Numéro de publication internationale: WO 2014/184390

(56) Documents cités:
- WO-A1-2012/131216
- WO-A2-03/022999
- US-A- 3 928 140
- US-A1- 2006 172 370
- US-A1- 2008 310 692
- US-B1- 7 016 523
- Buzalewicz I.: "Influence of various growth conditions on Fresnel diffraction patterns of bacteria colonies examined in the optical system with converging spherical wave illumination", OPTICS EXPRESS, vol. 19, no. 22, 24 October 2011 (2011-10-24), pages 21768-21785, XP055309883,

## Description

La présente invention concerne un procédé d'observation d'organismes. L'invention concerne également un système d'observation d'organismes associé.

L'invention s'inscrit dans le domaine de la microbiologie et, plus précisément, celui de l'identification d'organismes biologiques et de l'étude de l'interaction des organismes avec un milieu.

Il est connu de faire des cultures d'organismes sur des milieux particuliers. Ces milieux sont des milieux nutritifs gélosés. Ces milieux permettent d'augmenter le nombre d'organismes prélevés, soit de manière spécifique soit en obtenant une séparation spatiale des différentes types d'organismes composant le prélèvement. L'amplification de manière spécifique est obtenue par la présence d'inhibiteurs favorisant la croissance de certains organismes au détriment d'autres. L'isolement spatial de différents types d'organismes n'est obtenu que dans le cas d'un prélèvement polymicrobien.

Un des inconvénients de la culture sur de tels milieux est le temps impliqué pour obtenir à partir d'un organisme unique un ensemble d'organismes qui soit observable et prélevable par un opérateur humain. Ceci requiert généralement une vingtaine de générations, soit un temps relativement important d'au moins 24 heures.

Il existe donc un besoin pour un procédé d'observation d'organismes dans des milieux solides dont la mise en œuvre est plus rapide que les documents de l'état de la technique.

Selon l'invention, ce but est atteint par un procédé d'observation d'un échantillon, l'échantillon comprenant un ensemble d'organismes, un substrat solide supportant l'ensemble d'organismes. Le procédé comporte une étape d'illumination d'au moins une partie de l'échantillon par un faisceau lumineux. Le procédé comprend aussi une étape d'acquisition d'une première figure de diffraction correspondant à une image d'ondes issues de la diffraction du faisceau lumineux par une première partie de l'ensemble d'organismes. Le procédé comporte également une étape d'acquisition d'une deuxième figure de diffraction correspondant à une image d'ondes issues de la diffraction du faisceau lumineux par une deuxième partie de l'ensemble d'organismes. Le procédé comprend aussi une étape de comparaison de la deuxième figure de diffraction avec la première figure de diffraction et une étape de détermination d'au moins une caractéristique relative à l'ensemble d'organismes à partir du résultat de l'étape de comparaison. Le procédé comprend, lors de chaque étape d'acquisition, une étape d'adaptation de la taille du faisceau lumineux par rapport à la taille de la partie de l'ensemble d'organismes considérée, l'ensemble d'organismes étant situé dans un plan et chaque étape adaptation de la taille du faisceau lumineux étant mise en œuvre dans le plan pour que le rayon du faisceau lumineux dans le plan soit supérieur ou égal à 0,5 fois la taille de la partie de l'ensemble d'organismes considérée et soit inférieur ou égal à 5 fois la taille de la partie de l'ensemble d'organismes considérée.

Suivant des modes de réalisation particuliers, le procédé comprend une ou plusieurs des caractéristiques suivantes, prise(s) isolément ou suivant toute combinaison techniquement possible :
- chaque étape d'adaptation de la taille du faisceau lumineux est mise en œuvre dans le plan pour que le rayon du faisceau lumineux dans le plan soit supérieur ou égal à 1 fois la taille de la partie de l'ensemble d'organismes considérée, de préférence supérieur ou égal à 3 fois la taille de la partie de l'ensemble d'organismes considérée.
- la première figure de diffraction est acquise à un instant différent de la deuxième figure de diffraction.
- la première partie et la deuxième partie sont distinctes.
- l'ensemble d'organismes est situé dans un plan et présente une extension maximale selon au moins une direction, le rayon du faisceau lumineux dans le plan à l'étape d'illumination étant compris entre une et cinq fois l'extension maximale de l'ensemble d'organismes.
- le substrat solide est propre à faire croître au moins une partie de l'ensemble d'organismes et, à chaque instant, le rayon du faisceau lumineux dans le plan à l'étape d'illumination est maintenu à une taille comprise entre trois et cinq fois l'extension maximale de l'ensemble d'organismes.
- le substrat solide est propre à faire croître au moins une partie de l'ensemble d'organismes et l'échantillon comprend une zone dans laquelle le substrat solide comprend un antibiotique, la deuxième figure de diffraction correspondant à une image d'ondes issues de la diffraction du faisceau lumineux par la zone de l'échantillon et la caractéristique déterminée à l'étape de détermination est la sensibilité de l'organisme à l'antibiotique.
- le substrat solide est propre à faire croître au moins une partie de l'ensemble d'organismes et l'échantillon comprend une zone dans laquelle le substrat solide comprend un antibiotique dont la concentration suit un gradient selon une direction. Le procédé comporte une étape d'acquisition d'une pluralité de figures de diffraction correspondant à une image d'ondes issues de la diffraction du faisceau lumineux par une pluralité de parties de la zone l'échantillon, la concentration en antibiotique dans chaque partie étant différente et une étape de comparaison de chaque image de la pluralité d'images avec la première image, la caractéristique déterminée à l'étape de détermination étant la concentration inhibitrice minimale de l'organisme à l'antibiotique.
- le substrat solide est propre à faire varier l'indice optique d'au moins une partie des organismes de l'ensemble d'organismes.
- le substrat solide est un substrat chromogène précipitant.

L'invention concerne aussi un système d'observation d'un échantillon, l'échantillon comprenant un ensemble d'organismes, un substrat solide supportant l'ensemble d'organismes, le système d'observation comprenant une source lumineuse adaptée à émettre un faisceau lumineux, un porte-échantillon adapté à accueillir l'échantillon de sorte que l'échantillon soit illuminé par le faisceau lumineux que la source lumineuse est adaptée à émettre et de sorte que la taille du faisceau lumineux soit adaptée par rapport à la taille de la partie de l'ensemble d'organismes considérée, l'ensemble d'organismes étant situé dans un plan, un dispositif de détection adapté à acquérir des figures de diffraction correspondant à une image d'ondes issues de la diffraction du faisceau lumineux par une partie de l'échantillon, une platine de déplacement solidaire du dispositif de détection et du porte-échantillon, la platine de déplacement étant déplaçable avec une amplitude de mouvement permettant d'adapter la taille du faisceau lumineux dans le plan pour que le rayon du faisceau lumineux dans le plan soit supérieur ou égal à 0,5 fois la taille de la partie de l'ensemble d'organismes considérée et soit inférieur ou égal à 5 fois la taille de la partie de l'ensemble d'organismes considérée, et un calculateur adapté à comparer des figures de diffraction acquises par le dispositif de détection et à déterminer au moins une caractéristique relative à l'ensemble d'organismes à partir du résultat de la comparaison.

D'autres caractéristiques et avantages de l'invention apparaîtront à la lecture de la description qui suit de modes de réalisation de l'invention, donnés à titre d'exemple uniquement et en référence aux dessins qui sont :
- figure 1, une vue schématique du système d'observation d'espèces biologiques selon l'invention ;
- figure 2, un graphique illustrant l'évolution du rayon du faisceau laser en fonction de la distance par rapport à l'échantillon de la figure 1 ;
- figures 3 à 10, des vues expérimentales d'une première expérience de mise en œuvre du procédé d'observation d'organismes selon un premier mode de réalisation de l'invention ;
- figures 11 à 18, des vues expérimentales d'une deuxième expérience de mise en œuvre du procédé d'observation d'organismes selon un premier mode de réalisation de l'invention ;
- figures 19 et 20, des représentations des nuages de points obtenus par une analyse en composante principale menée sur des figures de diffraction obtenues dans le cadre d'une troisième expérience de mise en œuvre du procédé d'observation d'organismes selon un premier mode de réalisation de l'invention ;
- figure 21, une représentation des nuages de points obtenus par une analyse en composante principale menée sur des figures de diffraction obtenues dans le cadre d'une quatrième expérience de mise en œuvre de procédé d'observation d'organismes selon un premier mode de réalisation de l'invention ;
- figures 22 à 25, des vues de figures de diffraction obtenues par mise en œuvre du procédé d'observation d'espèces biologiques selon un deuxième mode de réalisation de l'invention.

Dans la suite, il est défini les notions de « amont » et « aval » en référence au sens de propagation de la lumière.

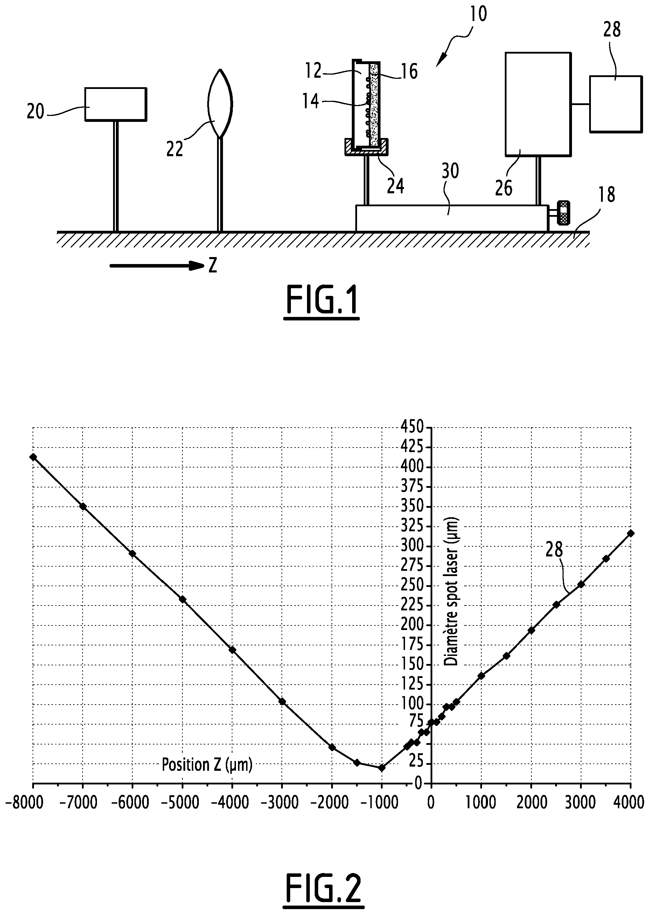

Le système d'observation 10 présenté à la figure 1 permet l'observation d'un échantillon 12.

L'échantillon 12 comprend un ensemble d'organismes 14 et un substrat solide 16 supportant l'ensemble d'organismes 14.

Selon les cas, un organisme de l'ensemble d'organismes 14 est acellulaire ou cellulaire. Parmi les organismes cellulaires, sont usuellement distingués les organismes procaryotes et les organismes eucaryotes. Les archées (aussi appelées archaebactéries) et les bactéries sont des exemples d'organisme procaryote. Les organismes eucaryotes sont soit unicellulaires, soit pluricellulaires. A titre d'exemple, les protozoaires, les amibes, les algues ou les levures sont des organismes eucaryotes unicellulaires. Les organismes eucaryotes pluricellulaires sont des cellules issues, par exemple, de mammifères humain et non-humain, de champignons, de végétaux, de protistes ou de chromistes.

Par organismes, il est entendu par exemple des microorganismes, en particulier des bactéries accolées les unes aux autres, de façon à former des colonies bactériennes. Ainsi, les organismes sont répartis sur le substrat 16 en différentes parties, chaque partie correspondant à un organisme ou à une agglomération d'organismes. Chaque partie peut notamment correspondre à une colonie bactérienne. Il est considéré, pour la suite, que l'ensemble d'organismes 14 est situé dans un plan perpendiculaire à un axe horizontal Z.

En outre, dans ce plan, l'ensemble d'organismes 14 présente une extension maximale selon une direction. Par extension maximale, il est entendu une taille caractéristique de l'ensemble d'organismes 14. Il s'agit par exemple du diamètre du cercle dans lequel est inscrit l'ensemble d'organismes 14. D'une façon générale, l'invention s'applique à des organismes ou des ensembles d'organismes 14 dont l'extension maximale est comprise entre 100 nanomètres (nm) et 1 millimètre (mm), de préférence entre 1 micromètre (µm) et 100 µm.

Ainsi, l'invention concerne en particulier l'observation de colonies bactériennes de taille micrométrique, dont la taille caractéristique est inférieure à 1 mm, par exemple comprise entre 100 µm et 1 mm, voire entre 50 µm et 500 µm. Cela permet une observation à un stade précoce du développement des colonies. Il est alors gagné du temps par rapport aux méthodes basées sur l'observation de colonies bactériennes de taille millimétrique.

Le substrat solide 16 est propre à faire croître au moins une partie de l'ensemble d'organismes 14. Ainsi, le substrat 16 est lui-même un milieu de culture ou est placé au contact d'un milieu de culture.

Le substrat solide 16 est, par exemple, un milieu gélosé.

A titre d'exemple, le substrat solide 16 est un milieu Muller-Hinton 2 agar (noté milieu MH2 agar dans la suite de la description). Un tel milieu est adapté pour la réalisation d'antibiogrammes.

Selon une variante, le substrat solide 16 sert uniquement de colle biologique.

A titre d'exemple, le substrat solide 16 comprend de la polylysine ou du collagène de type I.

Dans l'exemple de la figure 2, l'échantillon 12 se présente sous la forme d'une boîte de Pétri. Une boîte de Pétri a une forme cylindrique dont la base est un cercle.

Le système d'observation 10 est posé sur une table optique 18 assurant la stabilité du système d'observation 10.

Le système d'observation 10 comporte depuis l'amont vers l'aval, une source lumineuse 20, un système optique 22, un porte-échantillon 24 portant l'échantillon 12, un dispositif de détection 26 et un calculateur 28.

Selon l'exemple de la figure 1, la source lumineuse est une source laser 20 est propre à émettre un faisceau laser. Selon une variante, la source lumineuse 20 est une diode électroluminescente (souvent désigné par l'acronyme anglais de LED pour « Light Emitting Diode »).

Par exemple, la source laser 20 est une diode laser. Dans l'exemple de la figure 1, le faisceau laser émis par la source laser 20 a une longueur d'onde de 543,5 nm. De préférence, la source lumineuse 20 émet dans une plage de longueurs d'ondes comprises entre 250 nm et à 1200 nm. D'une façon générale la longueur d'onde doit être inférieure à l'extension maximale de l'objet observé, tout en permettant l'utilisation de moyens de détection usuels. Les longueurs d'onde dans le domaine du visible ou du proche infrarouge sont alors préférées. En variante, la longueur d'onde du faisceau laser est dans une bande de longueurs d'onde différentes. La longueur d'onde dépend notamment de l'organisme à observer et de sa sensibilité à une illumination par un faisceau laser.

En outre, le faisceau laser émis par la source laser 20 a une puissance inférieure à 10 milliWatts (mW), et de préférence inférieure à 100 microWatts (µW) pour éviter un échauffement des organismes lorsque les organismes sont illuminés par le faisceau laser.

Le système optique 22 est, selon l'exemple de la figure 1, une lentille.

Les caractéristiques du système optique 22 ainsi que son positionnement par rapport à la source laser 20 sont choisies pour que le rayon du faisceau laser dans le plan comportant l'ensemble d'organismes 14 soit compris entre une et cinq fois l'extension maximale de l'ensemble d'organismes.

De préférence, les caractéristiques du système optique 22 ainsi que son positionnement par rapport à la source laser 20 sont choisies pour que le rayon du faisceau laser dans le plan comportant l'ensemble d'organismes 14 soit égal à trois fois l'extension maximale de l'ensemble d'organismes 14. Il a été constaté que cela permet l'obtention d'images plus contrastées.

Dans le mode de réalisation de la figure 1, l'évolution spatiale du rayon du faisceau laser en sortie du système optique 22 est représentée sur la figure 2.

Le rayon d'un faisceau laser dans un plan normal à l'axe de propagation du faisceau laser est défini à partir du profil gaussien de l'intensité du faisceau laser dans le plan normal. Le rayon est défini comme la demi-largeur à 1/e² du profil d'intensité.

Dans le cas de la figure 2, l'intensité du faisceau laser a été mesurée par un analyseur de faisceau laser à différents endroits le long de l'axe Z.

Le plan Z=0 correspond au plan comprenant l'ensemble d'organismes 14.

Les différents points visibles sur la figure 2 sont reliés par une courbe 28 qui est une interpolation gaussienne des points mesurés. Cette interpolation montre que le waist du faisceau laser en sortie du système optique 22 est de 25 microns (µm) dans l'exemple de la figure 1.

Par définition, dans la suite de la description, l'expression « taille d'un faisceau lumineux » désigne le rayon du faisceau lumineux considéré dans le plan Z=0, le rayon étant défini tel que précédemment. Respectivement, l'expression « taille d'un faisceau lumineux » désigne le rayon du faisceau lumineux considéré dans le plan Z=0, le rayon étant défini tel que précédemment.

En outre, la taille faisceau laser dans le plan comportant l'ensemble d'organismes 14 est égal à 75 µm. Une telle courbe permet d'ajuster le diamètre du faisceau laser incident en fonction de l'extension maximale de l'objet observé. Cette dernière peut être déterminée par un dispositif d'imagerie annexe. Ainsi, de préférence, la taille de chaque objet observé est déterminée préalablement à son observation, de façon à adapter la taille du faisceau lumineux incident. Dans l'exemple considéré, le diamètre du faisceau laser est égal à trois fois ladite extension maximale. Un tel ajustement permet une adaptation du faisceau par rapport à l'extension maximale de l'objet observé, sachant que cette dernière peut évoluer avec le temps.

D'autre part, un tel ajustement est adapté à l'observation successive d'objets, de tailles différentes, disposés sur le même substrat 16.

Ainsi, d'une façon générale, le diamètre du faisceau lumineux incident peut être adaptée à la taille de l'objet observé.

Une telle adaptation permet d'avoir une figure de diffraction exploitable, quelle que soit la taille des objets observés. En effet, si la taille du faisceau lumineux incident est trop grande par rapport à l'objet observé, le signal de diffraction, spécifique à l'objet, est noyé dans le signal lumineux incident trop intense. A l'inverse, si la taille du faisceau est trop faible par rapport à la taille de l'objet, le rayonnement diffracté est trop peu intense. Aussi, comme précédemment indiqué, il est préférable d'adapter la taille du faisceau, de façon à ce que la taille du faisceau lumineux soit comprise entre 0,5 fois et 5 fois la taille de l'objet à caractériser. Préférentiellement, la taille du faisceau lumineux est adaptée pour que la taille du faisceau lumineux soit comprise entre 3 fois et 5 fois la taille de l'objet à caractériser.

D'une façon générale, chaque figure de diffraction est formée d'une zone centrale, autour de laquelle s'étendent des anneaux concentriques. Lorsque la taille du faisceau est adaptée comme précédemment décrit, les anneaux sont suffisamment nombreux et contrastés pour permettre une analyse précise de la figure de diffraction.

Dans le cas de colonies bactériennes, la taille du faisceau laser est de quelques dizaines de microns, voire moins de dix microns, pour pouvoir observer des colonies de faible taille, par exemple une taille comprise entre 50 µm et 500 µm. La taille du faisceau laser est de de quelques millimètres lorsqu'il est souhaité observer des colonies micrométriques plus importantes, dont la taille est inférieure à 1 mm.

Selon une variante, le système optique 22 est une lentille à focale variable, ce qui permet également cet ajustement.

Le porte-échantillon 24 permet de maintenir l'échantillon 12 perpendiculaire à l'axe Z, tout en le maintenant dans le faisceau laser émis par la source laser 20.

Le dispositif de détection 26 est, par exemple, une caméra CCD, CCD étant l'acronyme de l'anglais Charge-Coupled Device.

Le dispositif de détection 26 est adapté à acquérir une image d'ondes issues de la diffraction du faisceau laser émis par la source laser 20 par au moins une partie de l'échantillon 12.

On note qu'il n'y a pas d'optique de grossissement entre l'échantillon 12 et le dispositif de détection 26.

Dans l'invention, une figure de diffraction résulte de l'interférence de photons diffusés élastiquement par l'échantillon 12 observé.

Selon l'exemple de la figure 1, le dispositif de détection 26 et le porte-échantillon 24 sont solidaires d'une platine de déplacement 30 déplaçable dans une direction parallèle à l'axe Z.

La platine de déplacement 30 présente une amplitude de mouvement permettant d'assurer qu'à chaque instant, la taille du faisceau laser comprenant l'ensemble d'organismes 14 soit maintenu à une taille compris entre une et cinq fois l'extension maximale de l'ensemble d'organismes 14.

Le calculateur 28 est adapté à comparer des figures de diffraction acquises par le dispositif de détection 26 et à déterminer au moins une caractéristique relative à l'ensemble d'organismes 14 à partir du résultat de la comparaison.

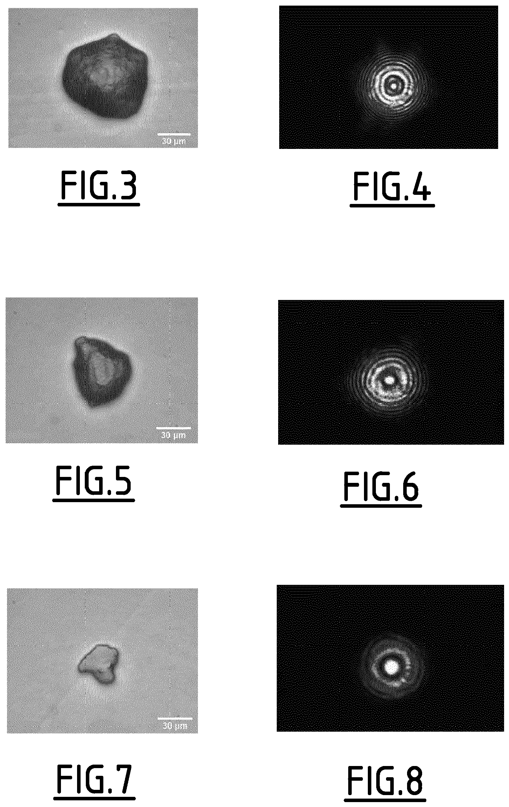

Le fonctionnement du système d'observation 10 va maintenant être décrit en référence à plusieurs expériences menées par la demanderesse et dont les résultats apparaissent notamment aux figures 3 à 25.

Les quatre premières expériences ont un protocole expérimental identique, seuls la manière d'analyser et les organismes choisis variant d'une expérience à l'autre.

Le substrat solide 16 est un milieu MH2 agar.

Selon les cas, le substrat solide 16 comporte un antibiotique en concentration croissante lorsqu'on s'éloigne du centre de la boîte de Pétri.

Par exemple, un tel gradient est obtenu par dépôt d'un disque imprégné de 30 microgrammes (µg) d'antibiotiques.

Le disque est, à titre d'illustration un disque de 6,5 mm de diamètre dont la référence est 66548 chez la société Biorad.

Lorsque le disque est déposé à la surface du substrat 16, un gradient de concentration d'antibiotique s'établit, dans le substrat 16, entre le disque et la périphérie du substrat 16.

L'antibiotique est de la gentamycine. La gentamycine est un antibiotique de la famille des aminosides ou aminoglycosides. Les antibiotiques de cette famille sont utilisés principalement dans le traitement d'infections impliquant des bactéries Gram - aérobies (*Pseudomonas, Acinetobacter* et *Enterobacter* par exemple).

Selon les cas, les organismes étudiés appartiennent à une souche sensible à la gentamycine ou résistante à la gentamycine.

La souche sensible est de l'espèce Escherichia coli de numéro ATCC 25922. Dans la suite de la description, la souche sensible est notée EC10.

La concentration inhibitrice minimale de la souche EC10 s'élève à 1,0 µg/mL. En microbiologie, la concentration inhibitrice minimale (aussi notée sous l'acronyme anglais MIC pour « minimum inhibitory concentration ») est la concentration la plus faible d'antimicrobien qui inhibe la croissance visible d'un organisme après une nuit d'incubation.

La valeur de 1,0 µg/mL pour la concentration inhibitrice minimale a été obtenu par réalisation d'un E-test®.

Le principe de l'E-test® est basé sur la combinaison des deux concepts : dilution et diffusion. Le système E-test® comprend une bande en plastique non poreuse calibrée par un gradient pré-établi en concentration d'antibiotiques couvrant 15 dilutions pour déterminer la MIC en µg/ml d'une souche testée en milieu gélosé.

La souche résistante est de l'espèce Escherichia coli de numéro ATCC 35421. Dans la suite de la description, la souche résistante est notée EC21.

La concentration inhibitrice minimale de la souche EC21 est supérieure à 256 µg/mL. Cette mesure a été obtenue par réalisation d'un E-test®.

Ainsi, on dispose d'une souche dite sensible (en l'occurrence EC10) et d'une couche dite résistante (en l'occurrence EC21).

Dans chaque cas, l'échantillon 12 à étudier est préparé selon le protocole suivant.

Au moins une cellule de la souche à étudier est cultivée sur un milieu gélosé Trypticase soja (souvent appelé sous l'acronyme TSA lié à sa dénomination anglaise de « Tryptic Soy Agar ») pendant moins de 24 heures.

Il est alors préparé une suspension de 5 millilitres (mL) comprenant de l'eau et une quantité de cellules de la souche telle que la turbidité de la solution soit égale à 0,5 McF (standard de McFarland).

La suspension obtenue est diluée au 1/1000^{ème} dans de l'eau en une seule étape. Dans les expériences considérées, 3 mL de la suspension sont versés dans 3 mL d'eau.

Un volume de 70 µL de la suspension diluée ainsi obtenue est alors étalé à l'aide d'un râteau sur le substrat solide 16 de MH2 agar. Un tel volume correspond à un étalement d'approximativement 1000 à 1500 colonies sur le substrat solide 16

Dans le cas où l'expérience est menée avec un substrat solide 16 comprenant le gradient d'antibiotiques, le disque est appliqué immédiatement après l'étape d'étalement.

L'ensemble de l'échantillon 12 est alors incubé pendant six heures à une température de 37 °C (Celsius).

Au bout de ces six heures, le système d'observation 10 est utilisé pour mettre en œuvre un procédé d'observation des cellules de l'échantillon 12.

Pour la première expérience, les organismes étudiés appartiennent à la souche EC10 et le substrat solide 16 comprend un gradient en gentamycine.

Dans un souci de clarté de l'illustration, pour chaque figure de diffraction enregistrée, l'image dans l'espace direct est également montrée, cette image n'étant pas utile pour mettre en œuvre le procédé d'observation selon l'invention.

Une première colonie de cellules de la souche EC10 de l'échantillon 12 est illuminée par le faisceau laser émis par la source laser 20. Le centre de cette colonie se trouve à une distance de 13,5 millimètres (mm) par rapport au centre du disque, dans une zone à faible concentration en gentamycine, car suffisamment éloignée du disque.

Selon l'exemple présenté, dans le plan de l'échantillon 12, la zone illuminée est trois fois plus importante que la zone occupée par les organismes observés.

L'image de la première colonie dans l'espace direct est visible sur la figure 3. La première colonie est assez développée en taille, la première colonie occupant un espace sensiblement circulaire de diamètre 60 µm environ. Ceci indique que le substrat solide 16 avec une faible concentration en gentamycine ne ralentit pas suffisamment la croissance des organismes.

Le dispositif de détection 26 acquiert alors une première figure de diffraction F1 correspondant à une image d'ondes issues de la diffraction du faisceau laser par la première colonie. La figure 4 illustre la figure de diffraction F1 obtenue.

Une deuxième colonie de cellules de la souche EC10 de l'échantillon 12 est alors illuminée par le faisceau laser émis par la source laser 20. Le centre de cette colonie se trouve à une distance de 9,1 mm par rapport au centre du disque, dans une zone avec une concentration en gentamycine supérieure à celle de la zone imagée dans le cas des figures 3 et 4.

L'image de la deuxième colonie dans l'espace direct est visible sur la figure 5. La deuxième colonie est moins développée en taille que celle de la figure 3, la taille maximale de la deuxième colonie étant de 50 µm environ. Ceci montre une sensibilité de l'organisme EC10 à cette concentration en gentamycine.

Le dispositif de détection 26 acquiert alors une deuxième figure de diffraction F2 correspondant à une image d'ondes issues de la diffraction du faisceau laser par la deuxième colonie. La figure 6 illustre la figure de diffraction F2 obtenue.

Une troisième colonie de cellules de la souche EC10 de l'échantillon 12 est alors illuminée par le faisceau laser émis par la source laser 20. Le centre de cette colonie se trouve à une distance de 8,3 mm par rapport au centre du disque, dans une zone avec une concentration en gentamycine supérieure à celle de la zone imagée dans le cas des figures 5 et 6.

L'image de la troisième colonie dans l'espace direct est visible sur la figure 7. La troisième colonie est moins développée en taille que celle de la figure 5, la taille maximale de la troisième colonie étant de 30 µm environ. Ceci montre une bonne sensibilité de l'organisme EC10 à cette concentration en gentamycine.

Le dispositif de détection 26 acquiert alors une troisième figure de diffraction F3 correspondant à une image d'ondes issues de la diffraction du faisceau laser par la troisième colonie. La figure 8 illustre la figure de diffraction F3 obtenue.

Une quatrième colonie de cellules de la souche EC10 de l'échantillon 12 est alors illuminée par le faisceau laser émis par la source laser 20. Le centre de cette colonie se trouve à une distance de 5,0 mm par rapport au centre du disque, dans une zone avec une concentration en gentamycine supérieure à celle de la zone imagée dans le cas des figures 7 et 8.

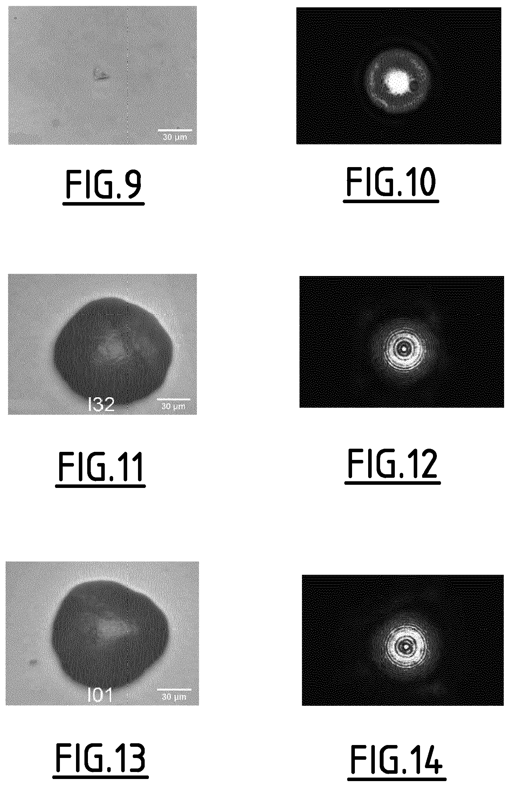

L'image de la quatrième colonie dans l'espace direct est visible sur la figure 9. La quatrième colonie est moins développée en taille que celle de la figure 7, la taille maximale de la quatrième colonie étant de 15 µm environ. Ceci montre une bonne sensibilité de l'organisme EC10 à cette concentration en gentamycine.

Le dispositif de détection 26 acquiert alors une quatrième figure de diffraction F4 correspondant à une image d'ondes issues de la diffraction du faisceau laser par la quatrième colonie. La figure 10 illustre la figure de diffraction F4 obtenue.

Ainsi, sur le même substrat solide, on observe des microcolonies dont le diamètre varie entre 15 µm et 60 µm. Comme précédemment décrit, si l'on souhaite disposer d'une figure de diffraction suffisamment exploitable pour chacun de ces objets, il convient d'adapter, lors de chaque observation, la taille du faisceau lumineux incident à la taille de chacun de ces objets, cette dernière étant préalablement déterminée.

Cette adaptation de taille est d'autant plus importante que lorsque les colonies bactériennes sont soumises à un antibiotique, les colonies bactériennes résistantes poursuivent leur croissance, tandis que les autres colonies subissent l'effet de l'antibiotique. On dispose donc, sur le même substrat, d'une variabilité en taille des objets à caractériser.

La première figure de diffraction F1 sert de figure de diffraction de référence.

Les deuxième, troisième et quatrième figures de diffraction F2, F3 et F4 sont respectivement comparées à la première figure de diffraction F1.

Selon un mode de réalisation, la comparaison est effectuée de manière visuelle par un observateur.

Lorsque la figure de diffraction correspond à une figure de diffraction, la comparaison porte notamment sur la largeur de la frange centrale, le nombre de franges observées ou la largeur des franges secondaires.

A titre d'exemple, en comparant la deuxième figure de diffraction F2 et la première figure de diffraction F1, il est observé en particulier que la frange centrale et la frange secondaire sont plus larges dans la deuxième figure de diffraction F2.

En comparant la quatrième figure de diffraction F4 et la première figure de diffraction F1, il est observé que la frange centrale et la frange secondaire sont plus larges dans la quatrième figure de diffraction F4.

Selon un autre mode de réalisation, la comparaison est effectuée à l'aide d'une décomposition de l'onde enregistrée par le dispositif d'acquisition 26 en polynômes de Zernike.

Chaque figure de diffraction F1, F2, F3 et F4 est ainsi décomposable en polynômes de Zernike.

De préférence, la décomposition en polynômes de Zernike est effectuée seulement sur les ordres détectables par le dispositif de détection 26. Cela permet de limiter le temps de calcul au moment de la décomposition de l'onde diffusée en polynômes de Zernike.

Les coefficients de la décomposition obtenue sont alors comparés lors de la comparaison.

A partir du résultat de la comparaison, au moins une caractéristique relative à l'ensemble d'organismes 14 est déterminée.

Dans le cadre de la première expérience proposée, deux caractéristiques sont déterminables : la sensibilité de l'organisme à la gentamycine c'est-à-dire la concentration à partir de laquelle la gentamycine a une action sur l'organisme.

Ainsi, le procédé d'observation permet d'observer des organismes pour en déduire des caractéristiques relatives aux organismes.

La première expérience montre comment déterminer la sensibilité de l'organisme à la gentamycine, voire la concentration à partir de laquelle la gentamycine a une action sur l'organisme lorsque la concentration de principe actif (en l'occurrence la gentamycine) à laquelle l'organisme est exposé est connue. La corrélation entre la distance au disque et la concentration en antibiotique peut notamment être déterminée sous la forme d'une droite de concordance. De manière similaire, il est possible d'identifier l'organisme ou d'obtenir une détection précoce de staphylocoques dorés résistants à la méticilline (aussi désignés sous l'acronyme MRSA), ou des bactéries de type VRE (acronyme de l'expression anglaise « Vancomycin Resistant Enterococci ») et ESBL (acronyme de l'expression anglaise « Extended Spectrum Beta Lactamase producing Enterobacteriaceae »).

Les caractéristiques relatives aux organismes sont obtenues en 6 heures par rapport à 48 heures pour les techniques de l'état de la technique. En effet, comme il est possible d'utiliser les figures de diffraction pour identifier les bactéries, la mise en œuvre de l'invention n'implique pas une étape de croissance de l'échantillon prélevé avant la réalisation de l'antibiogramme lui-même comme c'est le cas dans les méthodes de l'état de la technique.

Le procédé permet donc une détection rapide de caractéristiques relatives à des organismes.

Pour la deuxième expérience, il est réalisé la même expérience que la première expérience en remplaçant les cellules de la souche EC10 par les cellules de la souche EC21.

Les figures 11 à 18 illustrent la deuxième expérience.

Les figures 11 et 12 correspondent respectivement à l'observation en espace direct et de la figure de diffraction pour une distance de 7,29 mm du centre du disque.

Les figures 13 et 14 correspondent respectivement à l'observation en espace direct et de la figure de diffraction pour une distance de 6,63 mm du centre du disque.

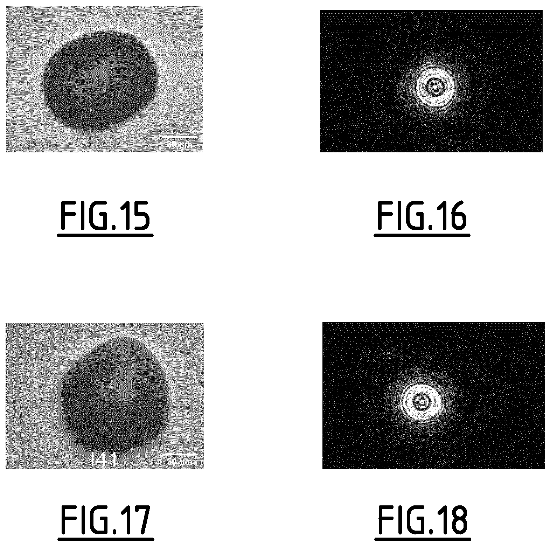

Les figures 15 et 16 correspondent respectivement à l'observation en espace direct et de la figure de diffraction pour une distance de 5,53 mm du centre du disque.

Les figures 17 et 18 correspondent respectivement à l'observation en espace direct et de la figure de diffraction pour une distance de 4,37 mm du centre du disque.

Contrairement au cas des figures de diffraction des figures 4, 6, 8 et 10 relatives à la première expérience, les figures de diffraction des figures 12, 14, 16 et 18 sont sensiblement identiques alors que la concentration en gentamycine est de plus en plus forte.

Cela montre bien que l'organisme EC21 est résistant à la gentamycine.

Cette observation est conforme à l'observation dans l'espace direct des figures 11, 13, 15 et 17. En effet, l'extension maximale de chaque colonie observée est de l'ordre de 90 µm. Ceci indique que le substrat solide 16 convient pour la croissance des organismes EC21 et que la gentamycine n'a aucun effet sur la croissance des organismes EC21.

La première expérience et la deuxième expérience présentées correspondent à un premier mode de réalisation dans lequel la première figure de diffraction correspond à une image d'ondes issues de la diffraction du faisceau laser par une première partie de l'échantillon 12 (en l'occurrence ici la première colonie) et la deuxième figure de diffraction correspond à une image d'ondes issues de la diffraction du faisceau laser par une deuxième partie de l'échantillon 12 (en l'occurrence ici la deuxième, la troisième ou la quatrième colonie). La première partie et la deuxième partie sont distinctes. En d'autres termes, dans chacune des deux expériences, quatre colonies sont réparties spatialement sur le disque. Cela rend possible des comparaisons de type spatiales.

Pour la troisième expérience, un nombre de figures de diffraction important est acquis (une centaine environ) pour des substrats solides 16 avec et sans gentamycine. L'organisme étudié est une cellule de la souche EC10.

En outre, l'étape de comparaison dans le cas de la troisième expérience est mise en œuvre à l'aide d'une analyse en composantes principales menée sur les figures de diffraction acquises au lieu de l'observation visuelle ou de l'analyse par décomposition en polynômes de Zernike suggérée en référence aux première et deuxième expérience.

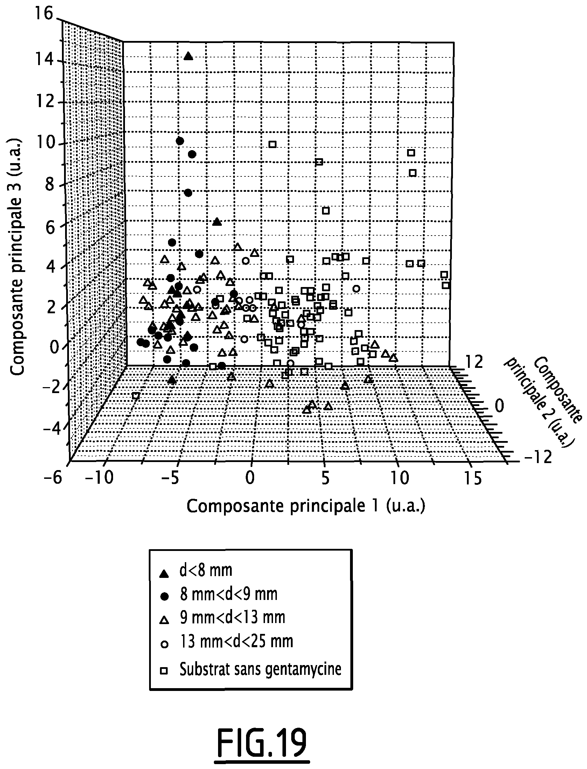

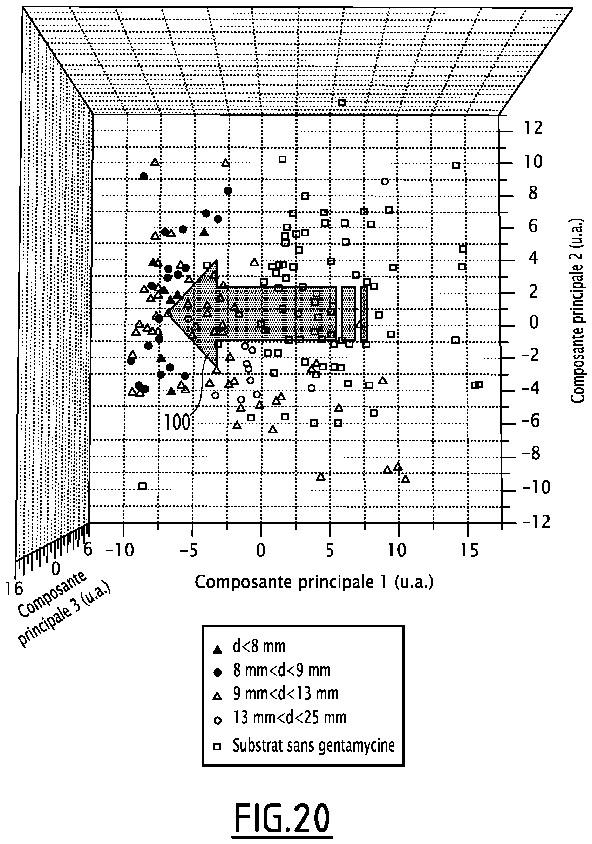

Le résultat de l'analyse en composantes principales obtenue est représenté sur les vues des figures 19 et 20.

Chaque point est représenté par trois coordonnées : sa valeur selon la première composante principale, sa valeur selon la deuxième composante principale et sa valeur selon la troisième composante principale.

Les points carrés correspondent aux expériences menées sur un substrat sans gentamycine.

Les points représentés par un cercle correspondent à une distance d de l'organisme imagé au centre du disque comprise entre 13 mm et 25 mm. Dans ce cas, la concentration en gentamycine est très inférieure à la concentration inhibitrice minimale de la souche EC10.

Les points représentés par un triangle correspondent à une distance d de l'organisme imagé au centre du disque comprise entre 9 mm et 13 mm. Dans ce cas, la concentration en gentamycine est inférieure à la concentration inhibitrice minimale de la souche EC10.

Les points représentés par un disque correspondent à une distance d de l'organisme imagé au centre du disque comprise entre 8 mm et 9 mm. Dans ce cas, la concentration en gentamycine est de l'ordre de la concentration inhibitrice minimale de la souche EC10.

Les points représentés par une pyramide correspondent à une distance d de l'organisme imagé au centre du disque inférieure à 8 mm. Dans ce cas, la concentration en gentamycine est supérieure à la concentration inhibitrice minimale de la souche EC10.

Il est observé que plus la concentration en gentamycine est importante, plus la valeur de la première composante principale est faible. C'est ce qu'indique la flèche 100 sur la figure 20. Cela correspond au fait que la souche EC10 est une souche sensible à la gentamycine.

Pour la troisième expérience, il est réalisé la même expérience que la troisième expérience en remplaçant les cellules de la souche EC10 par les cellules de la souche EC21.

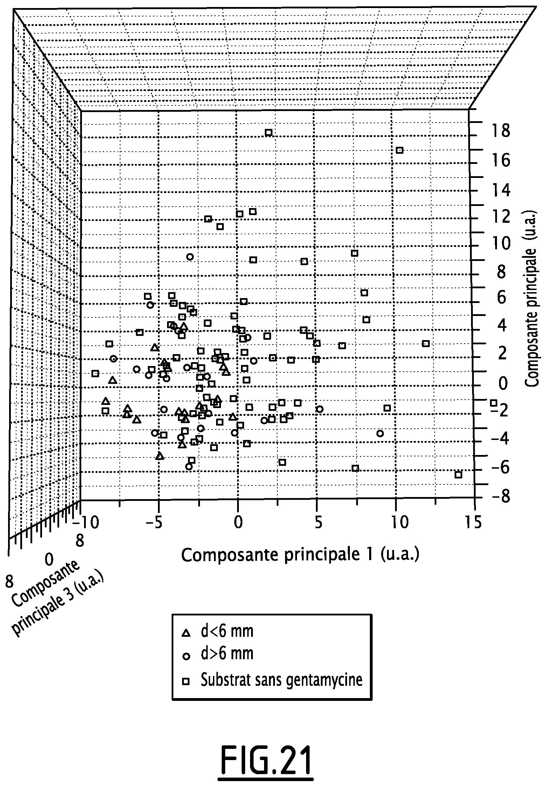

Le résultat de l'analyse en composantes principales obtenue est représenté sur la vue de la figure 21.

Les points carrés correspondent aux expériences menées sur un substrat sans gentamycine.

Les points représentés par un cercle correspondent à une distance d de l'organisme imagé au centre du disque supérieure à 6 mm. Les points représentés par un triangle correspondent à une distance d de l'organisme imagé au centre du disque inférieure à 6 mm

Dans le cas de la figure 21, il est observé que les nuages de points en présence et en absence d'antibiotique se recouvrent. Ceci correspond au fait que la souche E21 est résistante à la gentamycine.

Selon un deuxième mode de réalisation, chaque figure de diffraction acquise est une image du même organisme pris à des instants différents.

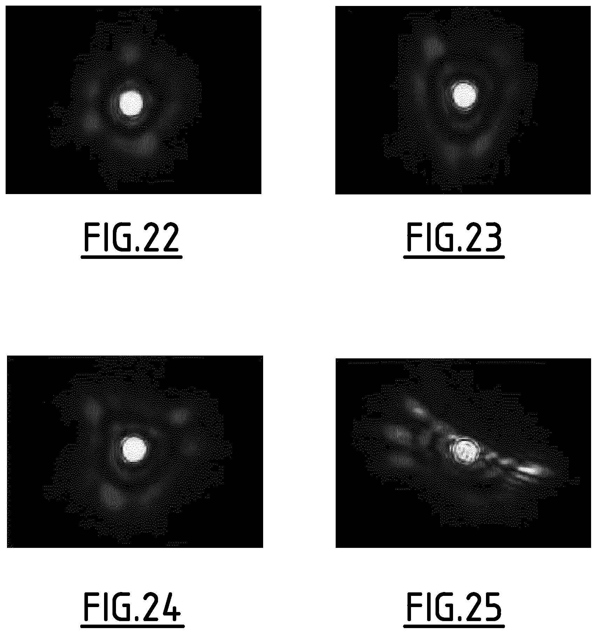

Les figures 22 à 25 illustrent ce cas, chaque figure de diffraction ayant été acquise à des instants successifs. Dans ce cas, le procédé est un procédé de comparaison temporelle.

La modification de la figure de diffraction est visible par comparaison de la figure de diffraction acquise à la première figure de diffraction acquise (la figure 22). La première figure de diffraction est alors la figure de référence.

Dans ce deuxième mode de réalisation, l'effet d'un antibiotique est mis en évidence dès qu'il inhibe la croissance de l'organisme étudié.

Le procédé de ce deuxième mode de réalisation est donc le plus rapide possible puisque, dès que l'antibiotique fait effet, le procédé permet d'obtenir l'information que l'organisme est sensible à l'antibiotique.

En variante, le substrat solide 16 est propre à faire varier l'indice optique d'au moins une partie des organismes de l'ensemble d'organismes 14. Cela permet d'accroitre les différences entre les différentes figures de diffraction.

Ainsi, à titre d'exemple le substrat solide 16 comporte des substrats chromogènes précipitants.

Un substrat indoxyle est un type de substrat chromogène précipitant. De tels substrats subissent une hydrolyse conduisant à des chromophores absorbants beaucoup à la longueur d'onde du laser employé pour la diffraction.

Le tableau 1 suivant donne plusieurs exemples de substrat chromogène précipitant susceptible d'être employé pour le procédé d'observation de l'invention.

De manière générale, tout substrat apte à générer par hydrolyse enzymatique un composé indoxyle ou quinoléine est susceptible d'être employé pour le procédé d'observation de l'invention. En outre, la forme du substrat (sel ou neutre) est indifférente pourvu que la capacité d'hydrolyse soit assurée.

Selon un mode de réalisation, la caractérisation de chaque figure de diffraction est précédée par une étape de localisation de l'objet à étudier et correspondant à ladite figure de diffraction. La localisation est, par exemple, réalisée en augmentant considérablement la taille du faisceau lumineux incident, de telle sorte que ce dernier éclaire une pluralité d'objets à caractériser, voire l'ensemble du substrat solide 16. La taille du faisceau lumineux est alors centimétrique.

Dans une telle configuration, selon les principes connus de l'imagerie sans lentille, chaque objet forme, à la surface du dispositif de détection 26, une figure de diffraction. Cette figure de diffraction ne permet pas une caractérisation précise, mais est suffisante pour localiser chaque objet dans le champ observé. On peut alors déterminer les coordonnées d'un objet, de façon à centrer le faisceau lumineux incident sur ce dernier, la taille du faisceau étant alors adaptée aux dimensions de l'objet.

Ainsi, lorsqu'une pluralité d'objets sont dispersés sur le substrat 16, on peut alternativement éclairer le susbtrat 16 à l'aide d'un faisceau lumineux permettant la formation d'une pluralité de figures de diffraction issues substrat 16 sur le dispositif de détection 26, chaque figure de diffraction correspondant à un objet, puis identifier la position d'une figure de diffraction et centrer le faisceau lumineux incident sur cette position, et réduire la taille du faisceau afin que la taille du faisceau soit adaptée à l'objet, c'est-à-dire comprise entre 0,5 fois et 5 fois la taille dudit objet (de préférence entre 1 fois et 5 fois la taille dudit objet), cela afin de former une figure de diffraction suffisamment précise pour permettre la caractérisation de l'objet.

**Table 1 : Pour chaque exemple de substrat chromogène précipitant, sont données successivement ses noms en français et en anglais ainsi que son numéro CAS (deuxième colonne), sa formule chimique (troisième colonne) et l'enzyme sur laquelle le substrat est efficace (quatrième colonne). Les substrats formant de la quinoléine, comme le 8-hydroxyquinoléine-β-D-glucuronide en bas du tableau, nécessitent la présence d'un métal chélatant, comme des ions ferreux, pour induire la formation d'un produit coloré insoluble.**

| | **Nom du substrat** | **Formule** | **Enzyme** |
|---|---|---|---|
| FR | 3-4-cyclohexenoesculetine β-D-galactopyranoside | 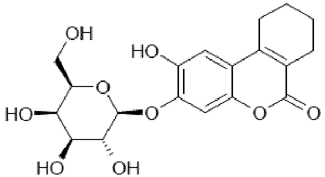 | β-galactosidase |
| GB | 3,4-Cyclohexenoesculetin β-D-galactopyranoside | | |
| CAS | 182805-65-8 | | |
| FR | Sel de sodium du 5-Bromo-4-chloro-3-indolyl β-D-glucuronide | 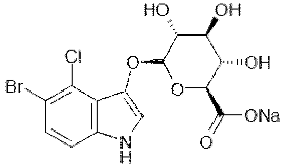 | glucuronidase |
| GB | 5-Bromo-4-chloro-3-indolyl β-D-glucuronide sodium salt | | |
| CAS | 129541-41-9 | | |
| FR | acétate de 5-bromo-4-chloro-3-indolyle | 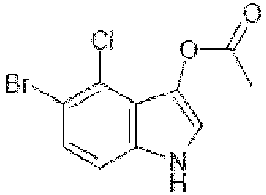 | estérase |
| GB | 5-bromo-4-chloro-3-indolyl acetate | | |
| CAS | 3252-36-6 | | |
| FR | 5-bromo-4-chloro-3-indolyl butyrate | 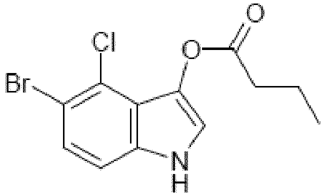 | lipase |
| GB | 5-Bromo-4-chloro-3-indolyl butyrate | | |
| CAS: | 129541-43-1 | | |
| FR | Sel de disodium du 5-bromo-4-chloro-3-indolyl phosphate | 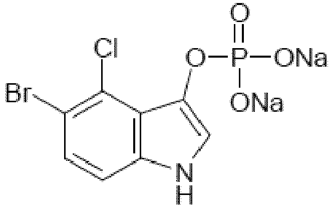 | phosphatase |
| GB | 5-Bromo-4-chloro-3-indolyl phosphate disodium salt | | |
| CAS: | 102185-33-1 | | |
| FR | 6-Chloro-3-indolyl α-D-galactopyranoside | 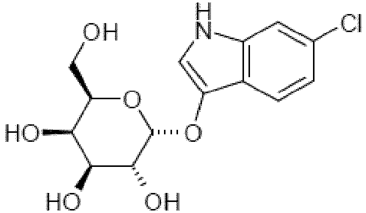 | galactosidase |
| GB | 6-Chloro-3-indolyl α-D-galactopyranoside | | |
| CAS | 198402-61-8 | | |
| FR | 6-Chloro-3-indolyl β-D-glucopyranoside | 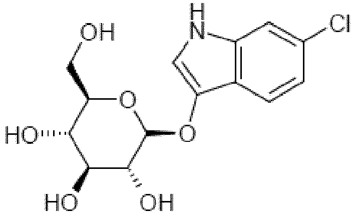 | glucosidase |
| GB | 6-Chloro-3-indolyl β-D-glucopyranoside | | |
| CAS | 159954-28-6 | | |

## Revendications

1. Procédé d'observation d'un échantillon (12), l'échantillon (12) comprenant :
- un ensemble d'organismes (14),
- un substrat solide (16) supportant l'ensemble d'organismes (14),
le procédé étant **caractérisé en ce que** le procédé comporte les étapes de :
- illumination d'au moins une partie de l'échantillon (12) par un faisceau lumineux,
- acquisition d'une première figure de diffraction (F1) correspondant à une image d'ondes issues de la diffraction du faisceau lumineux par une première partie de l'ensemble d'organismes (14),
- acquisition d'une deuxième figure de diffraction (F2) correspondant à une image d'ondes issues de la diffraction du faisceau lumineux par une deuxième partie de l'ensemble d'organismes (14),
- comparaison de la deuxième figure de diffraction (F2) avec la première figure de diffraction (F1),
- détermination d'au moins une caractéristique relative à l'ensemble d'organismes (14) à partir du résultat de l'étape de comparaison,
le procédé étant **caractérisé en ce que** le procédé comprend, lors de chaque étape d'acquisition, une étape d'adaptation de la taille du faisceau lumineux par rapport à la taille de la partie de l'ensemble d'organismes (14) considérée,
l'ensemble d'organismes (14) étant situé dans un plan et chaque étape d'adaptation de la taille du faisceau lumineux étant mise en œuvre dans le plan pour que le rayon du faisceau lumineux dans le plan soit supérieur ou égal à 0,5 fois la taille de la partie de l'ensemble d'organismes (14) considérée et soit inférieur ou égal à 5 fois la taille de la partie de l'ensemble d'organismes (14) considérée.

2. Procédé selon la revendication 1, dans lequel chaque étape d'adaptation de la taille du faisceau lumineux est mise en œuvre dans le plan pour que le rayon du faisceau lumineux dans le plan soit supérieur ou égal à 1 fois la taille de la partie de l'ensemble d'organismes (14) considérée, de préférence supérieur ou égal à 3 fois la taille de la partie de l'ensemble d'organismes (14) considérée.

3. Procédé selon la revendication 1 ou 2, dans lequel la première figure de diffraction (F1) est acquise à un instant différent de la deuxième figure de diffraction (F2).

4. Procédé selon la revendication 1 ou 2, dans lequel la première partie et la deuxième partie sont distinctes.

5. Procédé selon l'une quelconque des revendications 1 à 4, dans lequel l'ensemble d'organismes (14) est situé dans un plan et présente une extension maximale selon au moins une direction, le rayon du faisceau lumineux dans le plan à l'étape d'illumination étant compris entre une et cinq fois l'extension maximale de l'ensemble d'organismes (14).

6. Procédé selon la revendication 5, dans lequel le substrat solide (16) est propre à faire croître au moins une partie de l'ensemble d'organismes (14) et, à chaque instant, le rayon du faisceau lumineux dans le plan à l'étape d'illumination est maintenu à une taille comprise entre trois et cinq fois l'extension maximale de l'ensemble d'organismes (14).

7. Procédé selon l'une quelconque des revendications 1 à 6, dans lequel le substrat solide (16) est propre à faire croître au moins une partie de l'ensemble d'organismes (14) et dans lequel l'échantillon (12) comprend une zone dans laquelle le substrat solide (16) comprend un antibiotique, la deuxième figure de diffraction (F2) correspondant à une image d'ondes issues de la diffraction du faisceau lumineux par la zone de l'échantillon et la caractéristique déterminée à l'étape de détermination est la sensibilité de l'organisme à l'antibiotique.

8. Procédé selon l'une quelconque des revendications 1 à 7, dans lequel le substrat solide (16) est propre à faire croître au moins une partie de l'ensemble d'organismes et dans lequel l'échantillon (12) comprend une zone dans laquelle le substrat solide (16) comprend un antibiotique dont la concentration suit un gradient selon une direction, le procédé comportant une étape de :
- acquisition d'une pluralité de figures de diffraction correspondant à une image d'ondes issues de la diffraction du faisceau lumineux par une pluralité de parties de la zone l'échantillon, la concentration en antibiotique dans chaque partie étant différente,
- comparaison de chaque image de la pluralité d'images avec la première image,
la caractéristique déterminée à l'étape de détermination étant la concentration inhibitrice minimale de l'organisme à l'antibiotique.

9. Procédé selon l'une quelconque des revendications 1 à 8, dans lequel le substrat solide (16) est propre à faire varier l'indice optique d'au moins une partie des organismes de l'ensemble d'organismes (14).

10. Procédé selon la revendication 9, dans lequel le substrat solide (16) est un substrat chromogène précipitant.

11. Système d'observation (10) d'un échantillon (12), l'échantillon (12) comprenant :
- un ensemble d'organismes (14),
- un substrat solide (16) supportant l'ensemble d'organismes (14),
le système d'observation (10) comprenant :
- une source lumineuse (20) adaptée à émettre un faisceau lumineux,
- un porte-échantillon (24) adapté à accueillir l'échantillon (12) de sorte que l'échantillon (12) soit illuminé par le faisceau lumineux que la source lumineuse (20) est adaptée à émettre et de sorte que la taille du faisceau lumineux soit adaptée par rapport à la taille de la partie de l'ensemble d'organismes (14) considérée, l'ensemble d'organismes (14) étant situé dans un plan,
- un dispositif de détection (26) adapté à acquérir des figures de diffraction (F1) correspondant à une image d'ondes issues de la diffraction du faisceau lumineux par une partie de l'échantillon (12),
- un calculateur (28) adapté à comparer des figures de diffraction acquises par le dispositif de détection (26) et à déterminer au moins une caractéristique relative à l'ensemble d'organismes (14) à partir du résultat de la comparaison, **caractérisé en ce que** le système d'observation comprend :
- une platine de déplacement (30) solidaire du dispositif de détection (26) et du porte-échantillon (24), la platine de déplacement (30) étant déplaçable avec une amplitude de mouvement permettant d'adapter la taille du faisceau lumineux dans le plan pour que le rayon du faisceau lumineux dans le plan soit supérieur ou égal à 0,5 fois la taille de la partie de l'ensemble d'organismes (14) considérée et soit inférieur ou égal à 5 fois la taille de la partie de l'ensemble d'organismes (14) considérée.

## Patentansprüche

1. Verfahren zum Beobachten einer Probe (12), wobei die Probe (12) aufweist:
- eine Gruppe an Organismen (14),
- ein festes Substrat (16), welches die Gruppe an Organismen (14) trägt,
wobei das Verfahren **dadurch gekennzeichnet ist, dass** das Verfahren die Schritte aufweist des:
- Beleuchtens zumindest eines Teils der Probe (12) mittels eines Lichtstrahls,
- Erlangens eines ersten Beugungsbildes (F1), welches zu einem Bild von Wellen korrespondiert, welche von der Beugung des Lichtstrahls an einem ersten Teil der Gruppe an Organismen (14) stammen,
- Erlangens eines zweiten Beugungsbildes (F2), welches zu einem Bild von Wellen korrespondiert, welche von der Beugung des Lichtstrahls an einem zweiten Teil der Gruppe an Organismen (14) stammen,
- Vergleichens des zweiten Beugungsbildes (F2) mit dem ersten Beugungsbild (F1),
- Ermittelns mindestens einer Eigenschaft, welche die Gruppe an Organismen (14) betrifft, ausgehend vom Ergebnis des Schrittes des Vergleichens,
wobei das Verfahren **dadurch gekennzeichnet ist, dass** das Verfahren, während jedes Schrittes des Erlangens, einen Schritt des Anpassens der Größe des Lichtstrahls im Verhältnis zu der Größe des betrachteten Teils der Gruppe an Organismen (14) aufweist,
wobei sich die Gruppe an Organismen (14) in einer Ebene befindet und jeder Schritt des Anpassens der Größe des Lichtstrahls in der Ebene durchgeführt wird, damit der Radius des Lichtstrahls in der Ebene größer oder gleich 0,5 mal der Größe des betrachteten Teils der Gruppe an Organismen (14) ist und kleiner oder gleich 5 mal der Größe des betrachteten Teils der Gruppe an Organismen (14) ist.

2. Verfahren gemäß Anspruch 1, wobei jeder Schritt des Anpassens der Größe des Lichtstrahls in der Ebene durchgeführt wird, damit der Radius des Lichtstrahls in der Ebene größer oder gleich 1 mal der Größe des betrachteten Teils der Gruppe an Organismen (14) ist, vorzugsweise größer oder gleich 3 mal der Größe des betrachteten Teils der Gruppe an Organismen (14).

3. Verfahren gemäß Anspruch 1 oder 2, wobei das erste Beugungsbild (F1) zu einem anderen Zeitpunkt erlangt wird als das zweite Beugungsbild (F2).

4. Verfahren gemäß Anspruch 1 oder 2, wobei der erste Teil und der zweite Teil verschieden sind.

5. Verfahren gemäß irgendeinem der Ansprüche 1 bis 4, wobei sich die Gruppe an Organismen (14) in einer Ebene befindet und eine maximale Ausdehnung entlang mindestens einer Richtung hat, wobei der Radius des Lichtstrahls in der Ebene beim Schritt des Beleuchtens zwischen ein- und fünfmal der maximalen Ausdehnung der Gruppe an Organismen (14) liegt.

6. Verfahren gemäß Anspruch 5, wobei das feste Substrat (16) imstande ist, mindestens einen Teil der Gruppe an Organismen (14) wachsen zu lassen, und, zu jedem Zeitpunkt, der Radius des Lichtstrahls in der Ebene beim Schritt des Beleuchtens bei einer Größe beibehalten wird, welche zwischen drei- und fünfmal der maximalen Ausdehnung der Gruppe an Organismen (14) liegt.

7. Verfahren gemäß irgendeinem der Ansprüche 1 bis 6, wobei das feste Substrat (16) imstande ist, mindestens einen Teil der Gruppe an Organismen (14) wachsen zu lassen, und wobei die Probe (12) eine Zone aufweist, in welcher das feste Substrat (16) ein Antibiotikum aufweist, das zweite Beugungsbild (D2) zu einem Bild von Wellen korrespondiert, welche aus der Beugung des Lichtstrahls an der Zone der Probe stammen, und die Eigenschaft, welche im Schritt des Ermittelns ermittelt wird, die Empfindlichkeit des Organismus auf das Antibiotikum ist.

8. Verfahren gemäß irgendeinem der Ansprüche 1 bis 7, wobei das feste Substrat (16) imstande ist, mindestens einen Teil der Gruppe an Organismen wachsen zu lassen, und wobei die Probe (12) eine Zone aufweist, in welcher das feste Substrat (16) ein Antibiotikum aufweist, dessen Konzentration einem Gradienten entlang einer Richtung folgt, wobei das Verfahren einen Schritt aufweist des:
- Erlangens einer Mehrzahl von Beugungsbildern, welche zu einem Bild von Wellen korrespondieren, welche aus der Beugung des Lichtstrahls an einer Mehrzahl von Teilen der Zone der Probe stammen, wobei die Antibiotikumskonzentration in jedem Teil unterschiedlich ist,
- Vergleichens jedes Bildes der Mehrzahl von Bildern mit dem ersten Bild, wobei die Eigenschaft, welche im Schritt des Ermittelns ermittelt wird, die für den Organismus minimale hemmende Konzentration des Antibiotikums ist.

9. Verfahren gemäß irgendeinem der Ansprüche 1 bis 8, wobei das feste Substrat (16) imstande ist, den optischen Index zumindest eines Teils der Organismen der Gruppe an Organismen (14) variieren zu lassen.

10. Verfahren gemäß Anspruch 9, wobei das feste Substrat (16) ein präzipitierendes chromogenes Substrat ist.

11. System zum Beobachten (10) einer Probe (12), wobei die Probe (12) aufweist:
- eine Gruppe an Organismen (14),
- ein festes Substrat (16), welches die Gruppe an Organismen (14) trägt,
wobei das System zum Beobachten (10) aufweist:
- eine Lichtquelle (20), welche angepasst ist, um einen Lichtstrahl zu emittieren,
- einen Probenhalter (24), welcher angepasst ist, um die Probe (12) aufzunehmen, sodass die Probe (12) mittels des Lichtstrahls beleuchtet wird, den die Lichtquelle (20) zu emittieren angepasst ist, und sodass die Größe des Lichtstrahls angepasst ist im Verhältnis zu der Größe des betrachteten Teils der Gruppe an Organismen (14), wobei sich die Gruppe an Organismen (14) in einer Ebene befindet,
- eine Vorrichtung zum Detektieren (28), welche angepasst ist, um Beugungsbilder (F1) zu erlangen, welche zu einem Bild von Wellen korrespondieren, welche aus der Beugung des Lichtstrahls an einem Teil der Probe (12) stammen,
- einen Rechner (28), welcher angepasst ist, um die mittels der Vorrichtung zum Detektieren (26) erlangten Beugungsbilder zu vergleichen und mindestens eine Eigenschaft, welche die Gruppe an Organismen (14) betrifft, ausgehend vom Ergebnis des Vergleichs zu ermitteln,
**dadurch gekennzeichnet, dass** das System zum Beobachten aufweist:
- eine Platte zum Verschieben (30), welche mit der Vorrichtung zum Detektieren (26) und mit dem Probenhalter (24) fest verbunden ist, wobei die Platte zum Verschieben (30) mit einer Bewegungsamplitude verschiebbar ist, welche es erlaubt, die Größe des Lichtstrahls in der Ebene anzupassen, damit der Radius des Lichtstrahls in der Ebene größer oder gleich 0,5 mal der Größe des betrachteten Teils der Gruppe an Organismen (14) ist und kleiner oder gleich 5 mal der Größe des betrachteten Teils der Gruppe an Organismen (14) ist.

## Claims

1. A method for observing a sample (12), the sample (12) comprising:
- a set of organisms (14),
- a solid substrate (16) supporting the set of organisms (14),
the method being **characterized in that** the method includes the steps for:
- illuminating at least one portion of the sample (12) with a light beam,
- acquiring a first diffraction pattern (F1) corresponding to an image of waves from the diffraction of the light beam by a first portion of the set of organisms (14),
- acquiring a second diffraction pattern (F2) corresponding to an image of waves from the diffraction of the light beam by a second portion of the set of organisms (14),
- comparing the second diffraction pattern (F2) with the first diffraction pattern (F1),
- determining at least one characteristic relating to the set of organisms (14) from the result of the comparison step,
the method comprising, during each acquisition step, a step for adapting the size of the light beam relatively to the size of the relevant portion of the set of organisms (14),
the set of organisms (14) being located in a plane and each step for adapting the size of the light beam is applied in the plane so that the radius of the light beam in the plane is greater than or equal to 0.5 times the size of the relevant portion of the set of organisms (14) and less than or equal to 5 times the size of the relevant portion of the set of organisms (14).

2. The method according to claim 1, wherein each step for adapting the size of the light beam is applied in the plane so that the radius of the light beam in the plane is greater than or equal to once the size of the relevant portion of the set of organisms (14), preferably greater than or equal to 3 times the size of the relevant portion of the set of organisms (14).

3. The method according to claim 1 or 2, wherein the first diffraction pattern (F1) is acquired at a different instant from that of the second diffraction pattern (F2).

4. The method according to claim 1 or 2, wherein the first portion and the second portion are distinct.

5. The method according to any one of claims 1 to 4, wherein the set of organisms (14) is located in a plane and has a maximum extension along at least one direction, the radius of the light beam in the plane in the illumination step being comprised between one and five times the maximum extension of the set of organisms (14).

6. The method according to claim 5, wherein the solid substrate (16) is able to increase at least one portion of the set of organisms (14) and, at each instant, the radius of the light beam in the plane in the illumination step is maintained at a size comprised between three and five times the maximum extension of the set of organisms (14).

7. The method according to any one of claims 1 to 6, wherein the solid substrate (16) is able to increase at least one portion of the set of organisms (14) and wherein the sample (12) comprises an area in which the solid substrate (16) comprises an antibiotic, the second diffraction pattern (F2) corresponding to an image of waves from the diffraction of the light beam by the area of the sample and the characteristic determined in the determination step is the sensitivity of the organism to the antibiotic.

8. The method according to any one of claims 1 to 7, wherein the solid substrate (16) is able to increase at least one portion of the set of organisms and wherein the sample (12) comprises an area in which the solid substrate (16) comprises an antibiotic, for which the concentration follows a gradient along one direction, the method including a step for:
- acquiring a plurality of diffraction patterns corresponding to an image of waves from the diffraction of the light beam by a plurality of portions of the area of the sample, the antibiotic concentration in each portion being different,
- comparing each image of the plurality of images with the first image,
the characteristic determined in the determination step being the minimum inhibitory concentration of the organism to the antibiotic.

9. The method according to any one of claims 1 to 8, wherein the solid substrate (16) is able to vary the optical index of at least one portion of the organisms of the set of organisms (14).

10. The method according to claim 9, wherein the solid substrate (16) is a precipitating color-forming substrate.

11. A system (10) for observing a sample (12), the sample (12) comprising:
- a set of organisms (14),
- a solid substrate (16) supporting the set of organisms (14),
the observation system (10) comprising:
- a light source (20) adapted for emitting a light beam,
- a sample holder (24) adapted for receiving the sample (12), so that the sample (12) is illuminated by the light beam which the light source (20) is adapted to emit and so that the size of the light beam is adapted relatively to the size of the relevant portion of the set of organisms (14), the set of organisms (14) being located in a plane,
- a detection device (26) adapted for acquiring diffraction patterns (F1) corresponding to an image of waves from the diffraction of the light beam by a portion of the sample (12), and
- a computer (28) adapted for comparing the diffraction patterns acquired by the detection device (26) and for determining at least one characteristic relating to the set of organisms (14) from the result of the comparison,
**characterized in that** the system (10) for observing comprises:
- a displacement stage (30) integral with the detection device (26) and the sample holder (24), the displacement stage (30) being movable with a movement amplitude ensuring adapting the size of the light beam in the plane so that the radius of the light beam in the plane is greater than or equal to 0.5 times the size of the relevant portion of the set of organisms (14) and less than or equal to 5 times the size of the relevant portion of the set of organisms (14).
